# DEMANDE DE BREVET EUROPEEN

(11) **EP 4 219 505 A1**
(43) Date de publication de la demande: **02.08.2023**
(21) Numéro de dépôt: 23162719.1
(22) Date de dépôt: 16.03.2017
(51) Int. Cl.: C07D 493/04

(54) **PROCEDE DE FABRICATION DE DIANHYDROHEXITOL AVEC UNE ETAPE DE DISTILLATION SUR UN EVAPORATEUR A COUCHE MINCE**

(30) Priorité: 16.03.2016 FR 1652236
(62) Demande divisionnaire de: 17716565.1
(71) Demandeur: ROQUETTE FRERES, 62136 Lestrem (FR)
(72) Inventeur: WYART, Hervé, 62149 CUINCHY (FR); IBERT, Mathias, 59930 LA CHAPELLE D'ARMENTIERES (FR)
(74) Mandataire: Plasseraud IP

(57) **Abrégé**

La présente invention concerne un procédé de fabrication de 1,4:3,6-dianhydrohexitols comprenant les étapes de :
a) fourniture d'au moins un hexitol,
b) déshydratation dudit hexitol,
c) distillation du produit de déshydratation issu de l'étape b),
caractérisé en ce que :
- l'hexitol est fourni sous forme d'une solution aqueuse au niveau de l'étape a),
- la distillation est réalisée au moyen d'un évaporateur à couche mince au niveau de l'étape c).

## Description

La présente invention concerne un procédé de fabrication de 1,4:3,6-dianhydrohexitols, par fourniture d'une solution aqueuse d'au moins un sucre hydrogéné, déshydratation interne de celui-ci, et distillation du produit obtenu au moyen d'un évaporateur à couche mince. Dans toute la présente Demande, on utilisera de manière équivalente les expressions évaporateur « à couche mince », ou « à film mince » ou encore « à film raclé ».

Ce procédé se distingue des méthodes de l'art antérieur qui ont recours à la distillation comme étape unitaire de purification en mode batch qui conduisent à des rendements moindres et à des produits plus colorés. On obtient donc selon l'invention des produits esthétiquement plus acceptables et chimiquement plus stables. En outre, les techniques de distillation en mode batch recommandent souvent de mettre en oeuvre des produits facilitant ladite distillation ; ces derniers auxiliaires s'avèrent être dangereux pour l'homme ou néfastes pour l'environnement.

Le procédé selon l'invention s'écarte également de l'art antérieur en ce sens où il utilise comme produit de départ une solution aqueuse plutôt qu'une poudre : les produits pulvérulents occasionnent des problèmes de manipulation mais sont aussi source de danger, ils doivent par ailleurs être fondus ce qui a un impact économique majeur sur le procédé en question.

Valoriser nos ressources biologiques renouvelables est devenu un impératif écologique et économique majeur, face à l'épuisement et à la montée des prix des matières fossiles comme le pétrole. C'est dans ce contexte que s'inscrit le développement des 1,4:3,6-dianhydrohexitols.

Ces produits, également appelés isohexides, sont obtenus par déshydratation interne de sucres hydrogénés en C₆ (hexitols) tels que le sorbitol, le mannitol et l'iditol. Dans la présente demande, le terme « dianhydrohexitols » englobe l'isosorbide (1,4 - 3,6-dianhydrosorbitol), l'isomannide (1,4 - 3,6 dianhydro-mannitol), l'isoidide (1,4 - 3,6-dianhydro-iditol) de formules suivantes, ainsi que les mélanges de ces produits :

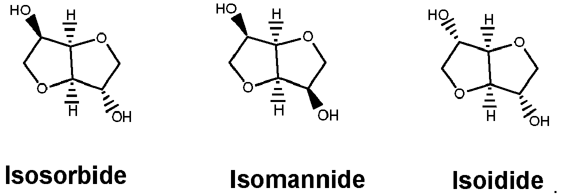

La Demanderesse indique que ces produits peuvent aussi être obtenus par déamination, selon la méthode décrite dans le document « Interconversion of DianhydroHexitols and of Saccharic Acids » (Nature, Oct 1, 1949, vol 464, pp. 573-574), une telle méthode restant pour le moins marginale dans le monde industriel.

Il existe aujourd'hui un fort potentiel de développement industriel pour les isohexides, notamment dans le domaine pharmaceutique, dans la fabrication des intermédiaires de synthèse chimique et dans le secteur des matières plastiques.

A titre d'exemple, le brevet GB 613,444 décrit l'obtention, par déshydratation en milieu eau / solvant, d'une composition d'isosorbide qui est ensuite soumise à un traitement de distillation puis de recristallisation dans un mélange alcool / éther.

Un traitement de purification associant distillation et recristallisation dans un alcool aliphatique inférieur (éthanol, méthanol) est également recommandé dans le brevet WO 00/14081. Ce document indique que dans le cas où la distillation est la seule étape de purification envisagée, il est avantageux de mettre en oeuvre du borohydrure de sodium.

D'autres auteurs ont également préconisé que l'étape de distillation soit menée en présence d'un composé boré tel que l'acide borique ou avec une résine anionique chargée en ions borate, comme décrit dans le brevet US 3,160,641.

Les brevets US 4,408,061 et EP 0 323 994 envisagent la mise en oeuvre de catalyseurs de déshydratation particuliers (respectivement un halogénure d'hydrogène gazeux et du fluorure d'hydrogène liquide), associés avantageusement à des acides carboxyliques comme co-catalyseurs puis la distillation des compositions brutes d'isosorbide ou d'isomannide ainsi obtenues.

Il résulte de ce qui précède que l'obtention de compositions à base d'isohexides impose généralement la mise en oeuvre, au moins à un moment donné, de moyens qui sont, pour le moins coûteux comme des halogénures d'hydrogène associés aux catalyseurs et cocatalyseur d'anhydrisation, soit potentiellement dangereux pour l'homme et l'environnement, en tout cas d'usage très réglementé, comme les solvants organiques. Ceci est d'autant plus préjudiciable que les techniques susmentionnées ont recours à la distillation en tant qu'étape finale de purification, distillation qui est très largement connue de l'homme du métier, disponible commercialement, facilement utilisable, et parfaitement adaptée à un usage industriel.

Des solutions ultérieures ont été proposées, en vue de fournir un procédé d'obtention de compositions contenant des isohexides, mettant en jeu une étape de distillation. On connaît à ce titre les demandes de brevet WO 2005 / 047228, WO 2013 / 169029, WO 2014 / 073843, WO 2014 / 129834 et KR 20140048436. Tous ces documents décrivent des procédés de fabrication d'isosorbide, où l'étape finale de purification est effectuée par distillation, au moyen d'un évaporateur à couche mince.

Ce type de dispositif est composé d'une partie cylindrique chauffée par double enveloppe, d'une partie supérieure servant à la séparation des vapeurs et d'un rotor tournant à grande vitesse. Le produit à traiter, introduit à la partie supérieure de la partie cylindrique, est réparti également sur la surface de chauffe par un anneau distributeur. Il est repris par les pales du rotor et étalé instantanément sur toute la paroi sous forme d'un film à grande turbulence. Le produit descend vers le pied de l'appareil en suivant le long de la paroi interne un mouvement hélicoïdal durant lequel l'évaporation des produits volatils a lieu. Les vapeurs qui se forment montent à contre-courant vers le haut de l'appareil et traversent le séparateur. Les gouttelettes et les mousses entraînées sont retenues et retombent dans la zone d'évaporation. Les vapeurs ainsi libérées des particules liquides passent dans un condenseur, dans une colonne ou tout autre étage de procédé suivant.

Les 5 demandes précitées, tout en mettant en oeuvre la technologie d'évaporation sur film mince, présentent également 2 autres caractéristiques communes : le produit initial est un sorbitol sous forme de poudre, et cette poudre est ensuite chauffée pour obtenir un fondu. La nature pulvérulente du sorbitol de départ n'est pas sans poser de nombreux problèmes : il est bien connu que les poudres présentent des risques d'explosion mais sont également difficiles à stocker et à transporter pour des questions de mottage. De plus, il est nécessaire de dépenser une importante quantité d'énergie de manière à transformer ladite poudre en fondu, ce qui ne va pas dans le sens d'un procédé économique.

On connaît enfin la demande de brevet WO 2009 / 126852. Elle décrit un procédé consistant à déshydrater du sorbitol sous vide et en présence d'un catalyseur acide. Selon une variante particulière, ledit sorbitol est présent sous forme d'une solution aqueuse avec une matière sèche comprise entre 40 % et 95 % de son poids total. Dans son exemple 7, elle décrit une synthèse d'isosorbide à partir d'une solution aqueuse de sorbitol contenant 93 % en poids sec de produit. La synthèse est suivie d'une distillation classique en mode batch. Néanmoins, la Demanderesse a su remarquer que dans un tel procédé, le produit final obtenu est caractérisé par une couleur jaune foncée. Or, il est bien connu que ces phénomènes de coloration traduisent la présence d'impuretés de diverses natures, impuretés qui peuvent nuire à la stabilité du produit mais aussi à son utilisation dans l'application finale.

Par conséquent, il n'existe pas à ce jour de méthode de fabrication de compositions d'isohexides, mettant avantageusement en oeuvre une étape simple de distillation continue en vue de purifier le produit final, qui soit exempte de composés nocifs pour l'homme et l'environnement, qui ne présente pas l'inconvénient de mettre en oeuvre un produit sous forme de poudre qui doit être fondu, et qui conduise à des produits finaux avec une coloration satisfaisante.

Poursuivant de nombreux travaux de recherche, la société demanderesse est parvenue à mettre au point une telle méthode. Celle-ci consiste en un procédé de fabrication de 1,4:3,6-dianhydrohexitols comprenant les étapes de :
a) fourniture d'au moins un hexitol,
b) déshydratation dudit hexitol,
c) distillation du produit de déshydratation issu de l'étape b),
caractérisé en ce que :
- l'hexitol est fourni sous forme d'une solution aqueuse au niveau de l'étape a),
- la distillation est réalisée au moyen d'un évaporateur à couche mince au niveau de l'étape c).

La première étape du procédé objet de la présente invention consiste à fournir au moins un hexitol, ledit hexitol étant fourni sous forme d'une solution aqueuse.

Cette étape est réalisée en mode continu.

De manière préférée, cette solution aqueuse présente une teneur en poids sec d'hexitol supérieure à 95 %, préférentiellement supérieure à 96 %, très préférentiellement supérieure ou égale à 97 % de son poids total, et inférieure à 99 % de son poids total.

Cette solution aqueuse est préférentiellement obtenue par élimination de l'eau d'une solution aqueuse initiale contenant au moins un hexitol, et la teneur en poids sec en ledit hexitol est comprise entre 40 % et 80 % de son poids total. L'élimination de l'eau est réalisée par tous moyens bien connus de l'homme du métier, notamment par chauffage, en particulier par distillation sous vide. La solution aqueuse initiale est par exemple un produit commercialisé par la demanderesse sous l'appellation NEOSORB.

La solution aqueuse contenant au moins un hexitol est aussi caractérisée en ce que l'hexitol est choisi parmi le sorbitol, le mannitol et l'iditol et leurs mélanges, et est préférentiellement le sorbitol.

La deuxième étape du procédé selon l'invention consiste à réaliser sur la solution contenant au moins un hexitol et fournie au cours de la première étape, la déshydratation dudit hexitol de manière à obtenir un 1,4:3,6-dianhydrohexitol.

Cette étape n'est aucunement limitante pour le procédé objet de la présente demande. Elle peut être réalisée selon un quelconque des procédés bien connus de l'homme du métier. A ce titre, on peut citer le brevet CA 1 178 288 qui rappelle en sa page 14, lignes 3-8 qu'il est recommandé de conduire la réaction de déshydratation proprement dite sous une atmosphère de gaz inerte pour éviter des réactions d'oxydation, notamment lorsque des températures et durées de réaction relativement importantes sont envisagées. Une variante selon la présente invention consiste donc à conduire cette étape de déshydratation sous une atmosphère de gaz inerte.

Le brevet US 4,861,513 décrit une réaction de déshydratation du sorbitol menée en présence simultanée de gaz inerte (azote) et d'un agent réducteur (hypophosphite de sodium) en vue de la préparation de mélanges de polyols particuliers, lesquels présentent une faible teneur (10 à 26 %) en dianhydrosorbitol. Pour sa part, le brevet GB 613,444 déjà évoqué décrit l'obtention, par déshydratation en milieu eau/solvant, d'une composition d'isosorbide qui est ensuite soumise à un traitement de distillation puis de recristallisation dans un mélange alcool/éther.

De manière préférée, les conditions de réalisation de cette étape de déshydratation sont les suivantes : la solution contenant l'hexitol obtenue lors de la première étape est introduite dans un réacteur. Simultanément, avant ou après l'introduction de la solution d'hexitol, le catalyseur de déshydratation est introduit dans le réacteur. Ce catalyseur peut être de tout type, tant qu'il permet la déshydratation de l'hexitol dans l'étape suivante. Ce catalyseur peut être un catalyseur hétérogène ou un catalyseur homogène. Il peut s'agir d'un catalyseur acide, en particulier d'un acide fort, ou de résines échangeuses d'ions, en particulier de résines échangeuses cationiques acides ou de catalyseurs de type zéolithe acide. Le catalyseur acide peut notamment être de l'acide sulfurique, de l'acide chlorhydrique, de l'acide para toluène sulfonique, de l'acide phosphorique, ou de l'acide méthane sulfonique. L'acide sulfurique est un catalyseur particulièrement préféré pour la fabrication de la composition selon l'invention.

La résine échangeuse cationique acide peut être une résine polystyrène sulfoné telle que la résine AG50W-X12 de BioRad. La zéolithe acide peut être une zéolithe béta.

Le catalyseur de déshydratation est introduit dans des quantités permettant la réalisation de l'étape de déshydratation. En particulier, lorsqu'on utilise l'acide sulfurique, on préfère utiliser des quantités inférieures à 2 % en masse par rapport à la masse totale d'hexitol, de préférence inférieure à 1,5 %, tout préférentiellement inférieure à 1,2 %.

L'étape de déshydratation peut se faire sous vide, sous flux d'un gaz inerte, par exemple de l'azote, ou encore sous pression en autoclave, ces trois méthodes permettant de faciliter l'élimination de l'eau et ainsi déplacer l'équilibre de la réaction.

Pour réaliser l'étape de déshydratation, il est nécessaire d'apporter de la chaleur au réacteur. Cette quantité de chaleur nécessaire dépend principalement de la nature et de la quantité de catalyseur utilisé et, dans une moindre mesure, des conditions de pression dans le réacteur lors de l'étape de déshydratation. Pour apporter la chaleur nécessaire, la température à l'intérieur du réacteur et sous laquelle est effectuée la réaction de déshydratation peut aller de 110 °C à 400 °C selon le catalyseur utilisé. Par exemple, lorsqu'on utilise, par rapport à la masse d'hexitol introduite, 1 % en masse d'acide sulfurique, on utilise de préférence une température supérieure ou égale à 135 °C, avantageusement supérieure ou égale à 150 °C. Avantageusement, la température reste inférieure à 300 °C.

A l'issue de l'étape de déshydratation, lorsque l'on utilise un catalyseur acide homogène, on réalise de préférence une étape de neutralisation du catalyseur.

Enfin, la troisième étape du procédé objet de la présente invention consiste à distiller le produit issu de l'étape précédente de déshydratation, c'est-à-dire le 1,4:3,6-dianhydrohexitol, la distillation étant conduite avec un évaporateur à film mince. Cette étape est réalisée dans des conditions de vide et de température permettant d'isoler l'isohexide du reste des constituants du produit.

Les conditions préférées de mise en oeuvre de cette étape sont les suivantes. Le produit issu de l'étape précédente de déshydratation est introduit en continu par l'intermédiaire d'une pompe dans la partie supérieure du corps cylindrique d'un évaporateur à film mince. Il est ensuite étalé sur les parois chauffées du corps cylindrique par les pales du rotor. La température de la double-enveloppe est maintenue entre 160 °C et 230°C, préférentiellement entre 170 °C et 220 °C. Le vide appliqué lors de l'opération de distillation est inférieur à 50 mbar, préférentiellement inférieur à 20 mbar, très préférentiellement inférieur à 10 mbar.

La coloration Gardner du 1,4:3,6-dianhydrohexitol obtenu après distillation, telle que mesurée sur le produit fondu dans une cellule de 1 cm de trajet optique à l'aide d'un colorimètre Lovibond PFXi-195/1, est inférieure au égale à 4, en particulier inférieure ou égale à 3, plus particulièrement inférieure ou égale à 2.5 , plus particulièrement inférieure ou égale à 2.1.

### EXEMPLES

### Exemple 1 selon l'invention

Dans un réacteur en verre de marque Schott, de contenance 1 litre, muni d'une double-enveloppe alimentée par un bain thermostaté à circulation d'huile, d'une pâle d'agitation, d'un thermomètre, d'une tête de distillation associée à un réfrigérant et à une recette de distillation, on introduit 1 kg d'une solution de sorbitol à 70 % de matière sèche (soit 700 g sec) commercialisée par la Demanderesse sous l'appellation NEOSORB^{®} 70/02. L'ensemble du montage est alors relié à une pompe à vide munie d'un vacuuomètre. L'agitation est mise en fonctionnement à 650 tr / min. La solution de sorbitol est ensuite chauffée sous vide (120 °C - 100 mbar) afin de distiller l'eau présente dans la solution. Après distillation de 279 g d'eau, on obtient 721 g d'une solution de sorbitol à 97 % de matière sèche. On ajoute 7 g d'acide sulfurique concentré, puis le mélange obtenu est chauffé sous vide (pression d'environ 100 mbars) à 150°C pendant 3 heures de façon à réaliser la réaction de déshydratation du sorbitol.

Le brut réactionnel est ensuite refroidi vers 100 °C puis neutralisé avec 11,4 g d'une solution de soude à 50 %. On obtient 575 g d'une composition neutralisée d'isohexide contenant 71,5 % soit 411 g d'isosorbide. La composition obtenue est ensuite introduite en 2 heures par l'intermédiaire d'une pompe à engrenage dans un évaporateur à film raclé en verre référence VDL 70-4 d'une surface d'évaporation de 0,05 m² dont la double-enveloppe est chauffée à 200 °C sous un vide de 5 mbar. On obtient 395 g d'isosorbide distillé, ce qui correspond à un rendement de distillation de 96,1%. La coloration Gardner de l'isosorbide obtenu après distillation est mesurée sur le produit fondu dans une cellule de 1 cm de trajet optique, à l'aide d'un colorimètre Lovibond PFXi-195/1. On obtient une valeur de coloration de 2.1 Gardner.

### Exemple 2

Dans un réacteur en verre de marque Schott, de contenance 1 litre, muni d'une double-enveloppe alimentée par un bain thermostaté à circulation d'huile, d'une pâle d'agitation, d'un thermomètre, d'une tête de distillation associée à un réfrigérant et à une recette de distillation, on introduit 1 kg d'une solution de sorbitol à 70 % de matière sèche (soit 700 g sec) commercialisée par la Demanderesse sous l'appellation NEOSORB^{®} 70/02. L'ensemble du montage est alors relié à une pompe à vide munie d'un vacuuomètre. L'agitation est mise en fonctionnement à 650 tr / min. La solution de sorbitol est ensuite chauffée sous vide (120 °C - 100 mbar) afin de distiller l'eau présente dans la solution. Après distillation de 279 g d'eau, on obtient 721 g d'une solution de sorbitol à 97 % de matière sèche. On ajoute 7 g d'acide sulfurique concentré, puis le mélange obtenu est chauffé sous vide (pression d'environ 100 mbars) à 150°C pendant 3 heures de façon à réaliser la réaction de déshydratation du sorbitol.

Le brut réactionnel est ensuite refroidi vers 100 °C puis neutralisé avec 11,4 g d'une solution de soude à 50 %. On obtient 575 g d'une composition neutralisée d'isohexide contenant 71,5 % soit 411 g d'isosorbide. On laisse la composition obtenue dans le réacteur en mettant la température de double-enveloppe à 200 °C et on applique un vide de 5 mbar pour effectuer la distillation. Dans ces conditions, on obtient 380 g d'isosorbide distillé, ce qui correspond à un rendement de distillation de 92,5 %. La coloration de l'isosorbide obtenu après distillation est de 4.8 Gardner.

### Exemple 3 selon l'invention

Dans un réacteur en verre de marque Schott, de contenance 1 litre, muni d'une double-enveloppe alimentée par un bain thermostaté à circulation d'huile, d'une pâle d'agitation, d'un thermomètre, d'une tête de distillation associée à un réfrigérant et à une recette de distillation, on introduit 1 kg d'une solution de sorbitol à 70 % de matière sèche (soit 700 g sec) commercialisée par la Demanderesse sous l'appellation NEOSORB^{®} 70/02. L'ensemble du montage est alors relié à une pompe à vide munie d'un vacuuomètre. L'agitation est mise en fonctionnement à 650 tr / min. La solution de sorbitol est ensuite chauffée sous vide (125 °C - 100 mbar) afin de distiller l'eau présente dans la solution. Après distillation de 285 g d'eau, on obtient 715 g d'une solution de sorbitol à 97.9 % de matière sèche. On ajoute 10.5 g d'acide méthane sulfonique concentré, puis le mélange obtenu est chauffé sous vide (pression d'environ 100 mbars) à 150°C pendant 3 heures de façon à réaliser la réaction de déshydratation du sorbitol.

Le brut réactionnel est ensuite refroidi vers 100 °C puis neutralisé avec 8.8 g d'une solution de soude à 50 %. On obtient 580 g d'une composition neutralisée d'isohexide contenant 70.8 % soit 410 g d'isosorbide. La composition obtenue est ensuite introduite en 2 heures par l'intermédiaire d'une pompe à engrenage dans un évaporateur à film raclé en verre référence VDL 70-4 d'une surface d'évaporation de 0,05 m² dont la double-enveloppe est chauffée à 180 °C sous un vide de 15 mbar. On obtient 372 g d'isosorbide distillé, ce qui correspond à un rendement de distillation de 90.7%. La coloration Gardner de l'isosorbide obtenu après distillation est mesurée sur le produit fondu dans une cellule de 1 cm de trajet optique, à l'aide d'un colorimètre Lovibond PFXi-195/1. On obtient une valeur de coloration de 1.6 Gardner.

### Exemple 4 selon l'invention

Dans un réacteur en verre de marque Schott, de contenance 1 litre, muni d'une double-enveloppe alimentée par un bain thermostaté à circulation d'huile, d'une pâle d'agitation, d'un thermomètre, d'une tête de distillation associée à un réfrigérant et à une recette de distillation, on introduit 1 kg d'une solution de sorbitol à 70 % de matière sèche (soit 700 g sec) commercialisée par la Demanderesse sous l'appellation NEOSORB^{®} 70/02. L'ensemble du montage est alors relié à une pompe à vide munie d'un vacuuomètre. L'agitation est mise en fonctionnement à 650 tr / min. La solution de sorbitol est ensuite chauffée sous vide (125 °C - 100 mbar) afin de distiller l'eau présente dans la solution. Après distillation de 285 g d'eau, on obtient 715 g d'une solution de sorbitol à 97.9 % de matière sèche. On ajoute 70 g de résine macroporeuse Amberlyst 36 dry, puis le mélange obtenu est chauffé sous vide (pression d'environ 100 mbars) à 150°C pendant 3 heures de façon à réaliser la réaction de déshydratation du sorbitol.

Le brut réactionnel est ensuite refroidi vers 100 °C, puis filtré pour séparer le milieu réactionnel de la résine. On obtient 568 g d'une composition d'isohexide contenant 72.0 % soit 409 g d'isosorbide. La composition obtenue est ensuite introduite en 2 heures par l'intermédiaire d'une pompe à engrenage dans un évaporateur à film raclé en verre référence VDL 70-4 d'une surface d'évaporation de 0,05 m² dont la double-enveloppe est chauffée à 190 °C sous un vide de 10 mbar. On obtient 384 g d'isosorbide distillé, ce qui correspond à un rendement de distillation de 93.8%. La coloration Gardner de l'isosorbide obtenu après distillation est mesurée sur le produit fondu dans une cellule de 1 cm de trajet optique, à l'aide d'un colorimètre Lovibond PFXi-195/1. On obtient une valeur de coloration de 1.9 Gardner.

Ces 4 essais illustrent donc bien la possibilité d'obtenir de l'isosorbide avec un procédé selon la présente invention.

On évite la mise en oeuvre de poudre en tant que produit de départ, ce qui exonère l'utilisateur des contraintes de manipulation de produits pulvérulents et élimine les risques d'explosion liés à l'emploi de ce type de produit.

Enfin, on améliore largement la coloration du produit final et ce, par rapport à des techniques en mode batch.

## Revendications

1. - Procédé de fabrication de 1,4:3,6-dianhydrohexitols comprenant les étapes de :
a) fourniture d'au moins un hexitol,
b) déshydratation dudit hexitol,
c) distillation du produit de déshydratation issu de l'étape b),
**caractérisé en ce que** :
- l'hexitol est fourni sous forme d'une solution aqueuse au niveau de l'étape a),
- la distillation est réalisée au moyen d'un évaporateur à couche mince au niveau de l'étape c).

2. - Procédé selon la revendication 1, **caractérisé en ce que** la solution aqueuse présente une teneur en poids sec d'hexitol supérieure à 95 %, préférentiellement supérieure à 96 %, très préférentiellement supérieure ou égale à 97 % de son poids total, et inférieure à 99 % de son poids total.

3. - Procédé selon une des revendications précédentes, **caractérisé en ce que** la solution aqueuse est obtenue par élimination de l'eau d'une solution aqueuse initiale contenant au moins un hexitol, et dont la teneur en poids sec en ledit hexitol est comprise entre 40 % et 80 % de son poids total.

4. - Procédé selon une des revendications précédentes, **caractérisé en ce que** pour la solution aqueuse contenant au moins un hexitol, ledit hexitol est choisi parmi le sorbitol, le mannitol et l'iditol et leurs mélanges, et est préférentiellement le sorbitol.

5. - Procédé selon une des revendications précédentes, **caractérisé en ce que** l'étape de déshydratation est conduite en présence d'un catalyseur de déshydratation.

6. - Procédé selon la revendication 5, **caractérisé en ce que** le catalyseur est un catalyseur du type résines échangeuses d'ions, en particulier de résines échangeuses cationiques acides ou de catalyseurs de type zéolithe acide.

7. - Procédé selon la revendication 5, **caractérisé en ce que** le catalyseur est un catalyseur acide choisi parmi l'acide sulfurique, l'acide chlorhydrique, l'acide para toluène sulfonique, l'acide phosphorique, l'acide méthane sulfonique et est préférentiellement l'acide sulfurique.

8. - Procédé selon une des revendications précédentes, **caractérisé en ce que** l'étape de déshydratation est conduite sous vide, sous flux d'un gaz inerte, ou sous pression en autoclave.

9. - Procédé selon une des revendications précédentes, **caractérisé en ce que** l'étape de déshydratation est conduite à une température de 110 °C à 400 °C.

10. - Procédé selon une des revendications précédentes, **caractérisé en ce que** l'étape de distillation est réalisée à une température comprise entre 160 °C et 230°C, préférentiellement entre 170 °C et 220 °C.

11. - Procédé selon une des revendications précédentes, **caractérisé en ce que** l'étape de distillation est réalisée sous un vide inférieur à 50 mbar, préférentiellement inférieur à 20 mbar, très préférentiellement inférieur à 10 mbar.
